# EUROPEAN PATENT APPLICATION

(11) **EP 0 583 945 A2**
(43) Date of publication of application: **23.02.1994**
(21) Application number: 93306367.9
(22) Date of filing: 12.08.1993
(51) Int. Cl.: G01N 33/82, G01N 33/60

(54) **Assay for 1,25 Dihydroxy Vitamin D**

(30) Priority: 14.08.1992 US 930570
(71) Applicant: WISCONSIN ALUMNI RESEARCH FOUNDATION, Madison, WI 53707-7365 (US)
(72) Inventor: Deluca, Hector F., Deerfield, Wisconsin 53531 (US); Koyama, Hidenori (n.m.i.), Osaka 590 (JP); Prahl, Jean M., Madison, Wisconsin 53719 (US); Uhland-Smith, Ann Uhland (n.m.i.), Madison, Wisconsin 53711 (US)
(74) Representative: Ellis-Jones, Patrick George Armine

(57) **Abstract**

An assay for 1,25-dihydroxy vitamin D is disclosed. The invention involves extracting blood serum using an organic solvent such as ethyl acetate, separating out potentially interfering other vitamin D metabolites using a silica column, and then adding pig receptor protein, radiolabeled 1,25-dihydroxy vitamin D, biotinylated antibody capable of binding to the receptor, and a facilitator protein such as BSA as part of an immunoprecipitation competitive binding assay. A kit for conducting this assay is also disclosed.

## Description

### Field Of The Invention

This invention relates to an assay for testing for the level of 1,25-dihydroxy vitamin D₃ in blood or blood component samples. More specifically it involves the use of both extraction and immunoassay techniques.

### Background Of The Invention

Vitamin D is a well known vitamin which has many useful functions in mammals. It is activated by 25-hydroxylation in the liver and subsequently by 1-hydroxylation in the kidney. This stimulates intestinal calcium transport, the mobilization of calcium from bone, and an increased reabsorption of calcium in the kidney. The production of the active form of vitamin D, 1,25-dihydroxy vitamin D (a/k/a D₃), is regulated by the need for calcium and phosphorus. Low serum calcium concentration stimulates the parathyroid gland to secrete parathyroid hormone, which in turn triggers the production of the "1,25-(OH)₂D" in the kidney. 1,25-(OH)₂D then directs the intestine to absorb calcium and phosphorus and the bone to mobilize calcium, and it stimulates renal reabsorption of calcium. These effects raise blood calcium to normal levels which in turn shuts down parathyroid secretion, shutting down production of 1,25-(OH)₂D. This metabolite also appears to play a major role in mineral and skeletal homeostasis.

Measurement of the level of 1,25-dihydroxy vitamin D in blood is therefore an important diagnostic tool with respect to certain diseases (e.g. kidney failure, osteoporosis). It is expected that it will also in the future provide useful research information.

Some competitive assays for this metabolite already exist. See S. Dokoh et al., 116 Anal. Biochem. 211-222 (1981); J. Eisman et al., 176 Arch. Biochem Biophys. 235-243 (1976); H. Perry et al., 112 Biochem. Biophys. Res. Commun. 431-436 (1983); R. Bouillon et al., 41 Ann. Endocrin. 435-36 (1980); R. Bouillon, 26 Clin. Chem. 562-567 (1980); R. Bouillon, 66 Eur. J. Biochem. 285-291 (1976); E. Mawer, et al., 190 Clin. Chim. Acta 199-209 (1990); S. Manolagas, et al., 56 J. Clin. Endocrinol. Metab. 751-760 (1983); P. Brumbaugh, et al., 13 Biochemistry 4091-4097 (1974); T. Reinhardt, et al., 58 J. Clin. Endocrinol. Metab. 91-98 (1984); R. Horst, et al., 38 Kidney Int. S28-S35 (1990).

In these prior art assays, mammalian blood serum or plasma was typically treated with an organic solvent that extracted vitamin D and its metabolites and left behind most of the interfering lipids. The extract was then pre-purified on a column. The semi-purified 1,25-(OH)₂D was then further purified by high performance liquid chromatography, yielding the purified 1,25-(OH)₂D. The HPLC purification was found to be a critical step when using the preferred intestinal source vitamin D receptor proteins. See e.g. J. Eisman et al., supra. Unfortunately, this HPLC step is time consuming and requires the use of relatively costly equipment.

After the HPLC, the isolated 1,25-(OH)₂D from serum was then added to a mixture of radiolabeled 1,25-(OH)₂D and either 1,25-(OH)₂D receptor which is a protein that specifically binds 1,25-(OH)₂D or an antibody raised to vitamin D metabolites. The unlabeled 1,25-(OH)₂D in the serum competed with the radiolabeled 1,25-(OH)₂D. The degree to which the binding of labeled 1,25-(OH)₂D was reduced by unlabeled 1,25-(OH)₂D was used to construct a standard curve to determine the amount of 1,25-(OH)₂D present in the sample. The level of bound, labeled 1,25-(OH)₂D was typically determined by absorbing the free or unbound labeled 1,25-(OH)₂D on dextran-coated charcoal. See e.g. J. Haddad et al., 33 J. Clin. Endocr. 992-995 (1971).

In recent years, several monoclonal antibodies (mAb) against the vitamin D receptor (VDR) have been developed. In U.S. patent 4,816,417, our laboratory reported on a new assay approach that involved using these antibodies to drag receptor out of solution after the receptor was used as a "fishing line" in a competitive binding assay between serum 1,25 vitamin D and labelled 1,25 vitamin D. This assay directly assayed the blood serum (without intermediate purifications). However, vitamin D transport protein was needed as a screening material to minimize interference from other vitamin D metabolites. While this immunoassay technique had advantages, it has now been discovered that in certain disease and other states blood samples can have widely varying levels of the required vitamin D transport protein. This causes some variability (absent the use of certain additional steps to correct for this variability).

Thus, it can be seen that the need exists for an improved immunoassay for 1,25-dihydroxy vitamin D₃.

### Summary Of The Invention

In one aspect, the invention provides a competitive binding assay to determine the presence of 1,25-dihydroxy vitamin D in a sample of animal blood, animal plasma, animal blood serum, or animal tissue. One exposes the sample to an organic solvent in which 1,25-dihydroxy vitamin D is soluble so as to extract the 1,25-dihydroxy vitamin D. One then adds to the extracted 1,25-dihydroxy vitamin D (a) receptor protein capable of binding to the 1,25-dihydroxy vitamin D, (b) labeled 1,25-dihydroxy vitamin D, and (c) antibody capable of binding to the receptor protein. One then measures the relative degree of binding of the labeled 1,25-dihydroxy vitamin D to receptor protein.

In a preferred form, prior to the addition step, the extracted portion is purified on a silica column to separate out interfering vitamin D metabolites. Then, during the addition step one also adds a fatty-acid free protein that does not bind with specificity to 1,25-dihydroxy vitamin D. The best results to date have been achieved where the added protein is bovine serum albumin or a cytosolic liver extract. Other proteins such as ovalbumin, cytochrome C, bacterial extract, yeast extract, or soybean protein should also work.

In another aspect, the inventor provides a competitive assay kit that includes labeled 1,25-dihydroxy vitamin D, receptor protein capable of binding to 1,25-dihydroxy vitamin D, antibody capable of binding to said receptor protein, and a facilitator protein such as BSA.

It will be appreciated that the invention provides a new assay in which the bound-free separation step is done using mAb for VDR, but which is not subject to variability due to differences in serum vitamin D transport protein levels. In this regard, along with potentially interfering lipids, the transport protein and many other serum proteins are removed by the extraction step.

It should be noted that a Sep-Pak silica column purification is preferably performed to remove potentially interfering vitamin D metabolites. Unlike HPLC, this type of chromatography can be conducted quickly and without expensive equipment. Other columns such as Sephadex LH20 or Lipidex 5000 can be used instead of the Sep-Pak to separate 1,25-dihydroxy vitamin D from 25-hydroxy vitamin D and 24,25-dihydroxy vitamin D.

Most surprisingly, it has been discovered that a facilitator protein must be added back or the mAb will not work. Preferred facilitators are bovine serum albumin and cytosic liver extract preparations. However, many fatty acid free proteins that do not bind specifically to 1,25-dihydroxy vitamin D should work. Note that adding back transport protein, which does specifically bind to the 1,25-dihydroxy vitamin D, will cause competition for the 1,25-(OH)₂D and thus interfere with the assay.

Note also that the sensitivity of this new assay is very high. This sensitivity, combined with the overall high recovery of 1,25-(OH)₂D₃ from serum after extraction and purification, indicates that human serum levels of 1,25-(OH)₂D₃ can be assayed in duplicate or triplicate with only 0.5 ml of serum sample.

In summary, we have developed an improved assay for detecting 1,25-(OH)₂D₃ using mAb against VDR, without requiring HPLC purification of the serum. This assay is useful for a rapid and precise determination of serum 1,25-(OH)₂D level from subjects with normal or diseased conditions, and will also be useful even after the administration of vitamin D metabolites to the subject.

An object of this invention therefor includes providing an immunoassay of the above kind in which 1,25-dihydroxy vitamin D can be assayed.

Another object is to provide an assay of the above kind which can be performed quickly, is relatively inexpensive, and is easy to perform without the use of costly equipment.

Another object is to provide kits for conducting assays of the above kind.

Still other objects and advantages of the present invention will be apparent from the description which follows. The preferred embodiments will be discussed below. These are merely examples of the invention. The claims should be examined in order to judge the full scope of the claims.

### Description Of The Preferred Embodiments

### Materials

Vitamin D compounds (1,25-(OH)₂D₃, 25-OH-D₃, 24,25-(OH)₂D₃) were gifts from either the Hoffmann-La Roche Company (Nutley, NJ) or Chugai Pharmaceuticals, Ltd. (Tokyo, Japan). 1,25-(OH)₂[26,27-³H]D₃ (166 Ci/mmol) was synthesized by Dupont/New England Nuclear (Boston, MA) as previously described J. Napoli et al., 19 Biochemistry 2515-2521 (1980) or purchased from Amersham International plc (Buckinghamshire, England). NSH-LC-Biotin was purchased from Pierce (Rockford, IL USA). Egg white avidin was from Calzyme Laboratory, Inc. (San Luis Obispo, CA). Sepharose CL-4B was from Pharmacia (Uppsala, Sweden). Bovine serum albumin (BSA; essentially free fatty acid free) was from Sigma (St. Louis, MO). Sep-Pak silica cartridge was purchased from Waters (Milford, MA). Buffers used as follows: TEDK₃₀₀: 50 mM Tris/HCl, 1.5 mM EDTA, 5 mM dithiothreitol, 300 mM KCl, pH 7.4. TE-Triton: 0.5% Triton X-100 in TE buffer (50 mM Tris/HCl, 1.5 mM EDTA, pH 7.4). PBS: 1.5 mM KH₂PO₄, 8.1 mM Na₂HPO₄, 137 mM NaCl, 2.7 mM KCl, pH 8.0. All organic solvents were liquid chromatography grade purified.

Pig intestinal nuclear extract containing abundant VDR and mAb XVIE6 or XVIE10, which specifically detects pig VDR, were prepared according to the method previously described. M. Dame et al., 25 Biochemistry 4523-4534 (1986). Note also that hybridomas capable of producing antibody to VDR have been deposited with the American Type Culture Collection, Rockville, Md., with A.T.C.C. #HB9496, and were previously made available in connection with U.S. patent 4,816,417. Availability of that deposit is not intended as a license.

Monoclonal antibodies dialyzed against 0.1 M sodium bicarbonate buffer, pH 8.3, were biotinylated by incubating 1 mg/ml of antibody solution with 60 µg of NHS-LC-biotin at room temperature for 4 h. Free biotin was removed by extensive dialysis against PBS. Avidin-Sepharose was synthesized as reported J. Kohm et al., 107 Biochem. Biophys. Res. 878-884 (1982), in which avidin was coupled to Sepharose CL-4B using a cyanogenbromide activation procedure. The VDR content in the intestinal nuclear extract was determined by a modified (M. Inaba et al., 1010 Biochem. Biophys. Acta 20-27 (1989)) hydroxylapatite assay. See W. Wecksler et al., 92 Anal. Biochem. 314-323 (1979). Protein concentrations were determined by the Bradford assay. M. Bradford, 72 Anal. Biochem. 248-254 (1976).

### Sample Extraction and Chromatography

To 0.5-ml of serum, 800 counts per minute of 1,25-(OH)₂-[³H]D₃ in 10 µl of ethanol was added for monitoring of recoveries. The sample was incubated for 1 h at room temperature to hydrolyze lipids, after which 0.5 ml of PBS and 3 vol. of dichloromethane were added. The mixture was shaken for 3 minutes at 3 oscillations per second. The lower organic phase (ethanol) was collected while the upper phase was re-extracted twice with 3 vol. of dichloromethane. The combined dichloromethane solution was evaporated under a stream of nitrogen gas, and the residue dissolved in 500 µl of 4% isopropanol/n-hexane. Sep-Pak Silica chromatography was done according to the method of T. Reinhardt et al., 58 J. Clin. Endocrinol. Metab. 91-98 (1984).

In brief, the sample was applied to a Sep-Pak Silica column preactivated with serial treatment of methanol, chloroform, n-hexane and 4% isopropanol/hexane. After the column was washed serially with 10 ml of 4% isopropanol/hexane and 8 ml of 6% isopropanol/hexane, the 1,25-(OH)₂D₃ was eluted with 10 ml of 15% isopropanol/hexane. Collected solvent was evaporated under a stream of nitrogen gas, and the residue dissolved in 70 µl of absolute ethanol, 20 µl of which was used to determine the recovery of tracer, and two 20 µl samples were applied to the radioreceptor assay.

### Alternative Extraction

An alternative extraction which conveniently results in the extracting solvent being the top layer is as follows. Serum samples (250 µL) were extracted three times with 500 µL of ethyl acetate. To determine percent recovery, 1,25-(OH)₂[³H]-D₃ was added to each of three additional samples, incubated at room temperature for 30 min and processed with the other samples. The organic layer was removed to small test tubes and dried under nitrogen in a 37°C water bath. The samples were then solubilized in 200 µL of 2% ethyl acetate in hexane for 30 min at room temperature.

Meanwhile Sep-Pak columns were prepared by rinsing one column for each sample-with hexane. The samples were then placed on the column and the test tubes and column reservoirs were rinsed three times with a few mL of 10% ethyl acetate in hexane. The columns were then washed with 20 mL of 10% ethylacetate in hexane followed by 20 mL of 20% ethylacetate in hexane. The 1,25-(OH)₂D₃ was eluted from the column using a solution of 50% ethylacetate in hexane and collected in large borosilicate tubes (18x150 mm). Greater than 90% of the tritiated compound was recovered in the 50% ethylacetate fraction in all assays performed. The samples were dried under nitrogen in a 37°C water bath and solubilized in 50 µL of ethanol. This solution was then subjected to analysis by immunoassay.

It should be appreciated that other organic solvents capable of extracting 1,25 vitamin D₃ (while leaving behind most of the lipids, proteins, and water soluable compounds) should be similarly useful. Further examples of extracting solvents are: diethylether, chloroform acetonitrile, butyl alcohol, benzene, and toluene.

### Titration Curve For Monoclonal Antibody And Avidin-Sepharose

Ten, 30 or 50 fmol of pig intestinal VDR was preincubated on ice for 4 h with 1.0 nM of [³H]-1,25-(OH)₂D₃ with or without 100-fold excess of 1,25-(OH)₂D₃. The indicated amounts of biotinylated mAb were added followed by incubation overnight at 0°C. Thereafter 100 µl of avidin-sepharose (50% slurry) was added, and the mixture was incubated for 1 h with continuous shaking at 0°C. The efficiency of the immunoprecipitation was assessed by a technique previously described. See Y. Lee et al., 264 J. Biol. Chem. 13701-13705 (1989). Titration of avidin-sepharose was done as described above using 20 fmol of VDR and 1 µg of biotinylated mAb.

### Non-equilibrium Radioreceptor Assay

Standard 1,25-(OH)₂D₃ (0.3-40 pg) or samples in 20 µl of ethanol were incubated for 1 h at room temperature with 270 µl of TEDK₃₀₀ solution, containing 20 fmol of pig intestinal VDR, 1 µg of biotinylated mAb, and 0.75 mg of BSA. The mixture was incubated for another 1 h at room temperature with 5000 cpm of [³H]-1,25-(OH)₂D₃ in 10 µl of ethanol and then maintained overnight on ice. Immunoprecipitation was done using saturating amounts of avidin-Sepharose. The Sepharose was washed 3 times with 750 µl of TE-Triton, transferred to scintillation vials, to which 4 ml of ACS-II (Amersham International plc) was added and the radioactivity was counted by scintillation counting. The 1,25-(OH)₂D₃ concentrations (picograms per ml) were determined from a standard curve and by correcting the values to 100% recovery.

### Studies

Sera was obtained after the overnight fasting from 14 normal adults, 16 patients with primary hyperparathyroidism, and 12 patients with chronic renal failure. Also, in 4 normal volunteers, 2.0 µg per day of 1α-OH-D₃ was administered orally for seven days, and blood was drawn before, 1 and 7 days after the treatment.

This assay was also applied to sera from control rats and those having chronic renal failure. Male Wistar rats (6 weeks old) with or without 5/6 nephrectomy were maintained for 16 weeks with ordinary diet. Thereafter, 0.15 µg/100 g body weight of 1,25-(OH)₂D₃ were administered intraperitoneally. At the indicated hours after treatment, blood was obtained from abdominal aorta under ether anesthesia.

### Results

Both dichloromethane extraction and ethyl acetate extraction were successfully used for the extraction of 1,25-(OH)₂D₃ from animal serum. Using the dichloromethane extraction and alkaline hydrolysis, interfering polar lipids were eliminated. When 0.25-0.5% of BSA was used in the radioreceptor area, standard curves for 1,25-(OH)₂D₃ with or without this extracted sample paralleled well, suggesting the absence of factor(s) that might interfere in the assay. Large amounts of 25-OH-D₃ in the dichloromethane extract which could competitively interfere with the assay were easily and quickly removed using the Sep-Pak silica cartridges.

It will be appreciated that while the extraction step removes potentially interfering lipids and certain potentially interfering proteins, applicants have discovered that it also removes certain proteins present in blood that are required if the intestinal receptor/mAb assay is to be used.

While BSA and cytosolic liver preparations have shown the best results to date, other proteins having the following characteristics should also work: fatty acid free, unlikely to specifically bind 1,25-(OH)₂ vitamin D₃, preferably of a molecular weight over 1000, and preferably water soluable or soluable in a dilute salt solution. The exact mechanism of the facilitation is unknown at this time, but based on results to date the effect is believed to be a combination of assisting in preventing the 1,25-dihydroxy vitamin D from sticking to the walls of the assay tube, stabilizing the receptor, and assisting the formation of the avidin-Sepharose complex.

## Claims

1. A competitive binding assay to determine the presence of 1,25-dihydroxy vitamin D in a sample of animal blood, animal plasma, animal blood serum, or animal tissue, comprising the steps of:
exposing the sample to an organic solvent that dissolves 1,25-dihydroxy vitamin D so as to extract the 1,25-dihydroxy vitamin D into the solvent;
adding to the extracted 1,25-dihydroxy vitamin D (a) receptor protein capable of binding to the 1,25-dihydroxy vitamin D, (b) labeled 1,25-dihydroxy vitamin D, and (c) antibody capable of binding to the receptor protein; and
measuring the relative degree of binding of the labeled 1,25-dihydroxy vitamin D to receptor protein.

2. The assay of claim 1, wherein prior to the measuring step there is an additional step of adding to the extracted 1,25-dihydroxy vitamin D a fatty-acid free protein that does not bind with specificity to 1,25-dihydroxy vitamin D.

3. The assay of claim 2, wherein the fatty acid free protein is albumin or cytosolic liver extract.

4. The assay of any one of claims 1 to 3 wherein the labeled 1,25-dihydroxy vitamin D is radiolabeled 1,25-dihydroxy vitamin D.

5. The assay of any one of the preceding claims wherein prior to the measuring step, receptor protein bound to said antibody is immuno-precipitated.

6. The assay of any one of the preceding claims wherein the organic solvent is ethyl acetate.

7. The assay of any one of claims 1 to 5, wherein the organic solvent is dichloromethane.

8. A competitive assay kit comprising labeled 1,25-dihydroxy vitamin D, receptor protein capable of binding to 1,25-dihydroxy vitamin D, antibody capable of binding to said receptor protein, and a fatty acid-free protein that does not bind with specificity to 1,25-dihydroxy vitamin D.

9. The kit of claim 8, wherein the labeled 1,25-dihydroxy vitamin D is radiolabeled.

10. The kit of claim 8 or 9 wherein the fatty acid-free protein is albumin or cytosolic liver extract.
